# EUROPÄISCHE PATENTSCHRIFT

(11) **EP 0 977 557 B1**
(45) Veröffentlichungstag und Bekanntmachung des Hinweises auf die Patenterteilung: **04.06.2003**
(21) Anmeldenummer: 98920539.8
(22) Anmeldetag: 20.04.1998
(51) Int. Cl.: A61K 9/16, A61K 31/60, A61K 47/32, A61K 9/28, A61P 1/00, A61P 35/00

(54) **PELLET-FORMULIERUNG ZUR BEHANDLUNG DES INTESTINALTRAKTES**
PELLET-TYPE FORMULATION INTENDED FOR TREATING THE INTESTINAL TRACT
FORMULATION DE TYPE PELLET, S'UTILISANT DANS LE TRAITEMENT DU TRACTUS INTESTINAL

(30) Priorität: 30.07.1997 DE 19732903
(43) Veröffentlichungstag der Anmeldung: 09.02.2000
(73) Patentinhaber: DR. FALK PHARMA GMBH, D-79108 Freiburg (DE)
(72) Erfinder: OTTERBECK, Norbert, D-88662 Überlingen (DE)
(74) Vertreter: Keller, Günter, Dr.
(86) Internationale Anmeldenummer: EP9802319
(87) Internationale Veröffentlichungsnummer: WO99006027

(56) Entgegenhaltungen:
- EP-A- 0 308 665
- EP-A- 0 671 168
- EP-A- 0 759 303
- WO-A-95/35100

## Beschreibung

Die vorliegende Erfindung betrifft eine Pellet-Formulierung zur Behandlung des Intestinaltraktes, die als pharmazeutischen Wirkstoff Aminosalicylsäure oder ein pharmazeutisch verträgliches Salz oder Derivate davon umfaßt.

Zur Behandlung intestinaler Erkrankungen, wie beispielsweise Colitis ulcerosa und Morbus Crohn, wird der Wirkstoff Aminosalicylsäure (insbesondere 5-ASA) oder dessen Derivate seit längerer Zeit erfolgreich verwendet (DE 31 51 196 A1).

Die Verwendung von 5-ASA und ihren Derivaten als chemotherapeutisches Mittel bei Dickdarmkrebs ist ebenfalls bekannt, wobei Polypen im Kolon und Rektum mit einem erhöhten Karzinomrisiko assoziiert werden (WO 95/18622).

Eine koloskopische Polypektomie bei Patienten mit Polypen im Kolon und/oder Rektum führt zu einer erheblichen Risikoreduktion für das Entstehen von Kolonkarzinomen und wird als Therapie insbesondere bei kolorektalen Polypen empfohlen. Die Rezidivrate nach Polypektomie ist jedoch hoch und beläuft sich auf etwa 6-30% pro Jahr. Aminosalicylsäure eignet sich zur längerfristigen Behandlung solcher Patienten und senkt die Rezidivrate kolorektaler Polypen.

Die Wirkung der Aminosalicylsäure bei der Behandlung intestinaler Erkrankungen, bzw. bei der Verhinderung ihres Wiederauftretens oder bei der Prävention daraus entstehender Folgeerkrankungen und möglicher Begleiterkrankungen erfolgt durch den Kontakt des Wirkstoffes direkt am Ort der Erkrankung im Darm, wobei die Wirkung der Aminosalicylsäure, oder einem Derivat davon, in direkter Relation zu seiner lokalen Konzentration am zu behandelnden Darmbereich steht.

Da entzündliche Prozesse oft größere Abschnitte des Intestinaltraktes befallen, sollte sich die Arzneiform reproduzierbar über weite Bereiche des Darmes ausbreiten und den Wirkstoff erst am Entzündungsort freisetzen.

Ein Problem bei der Behandlung mit Aminosalicylsäure besteht darin, daß der Wirkstoff sehr leicht resorbiert und über die Niere ausgeschieden wird, bevor seine Wirkung eintreten kann.

Im Stand der Technik sind Tabletten und Pellets bekannt, die mit einem magensaftresistenten Lack überzogen sind, um so eine frühzeitige Freigabe der Wirkstoffe zu verhindern.

Die FR-A 2 692 484 offenbart eine Tablette zur kontrollierten Freigabe von 4-ASA in einer hydrophilen Matrix, die aus quellbaren, eine Gelbarriere bildenden Polymeren besteht, und mit einem magensaftresistenten Überzug. Nach Auflösen des Überzuges quillt die Matrix auf und bildet eine Gelbarriere durch die der Wirkstoff heraus diffundiert. Die in der FR-A 2 692 484 offenbarte Zusammensetzung setzt den Wirkstoff im Darm nach einer ca. zweistündigen lag-Phase über eine Zeitdauer von weiteren 14 h in etwa linear frei.

Die EP 0 453 001 A1 offenbart eine pharmazeutische Zusammensetzung, worin der Wirkstoff mit mindestens zwei Membranen überzogen ist, von denen eine bei einem pH von ≽ 5,5 löslich ist und die andere bei diesem pH unlöslich aber für die Intestinalflüssigkeiten permeabel ist.

Die EP 0 148 811 A1 offenbart eine pharmazeutische Zusammensetzung, die aus einem Kern besteht, der den Wirkstoff enthält. Der Kern ist von zwei Schichten umgeben, von denen die innere Schicht eine Diffusionsmembran darstellt und die zweite Schicht ein anionisches Polymer und/oder eine Fettsäure mit einem pKₐ von 4,5 bis 7 ist.

Die EP 0 629 398 A1 offenbart pharmazeutische Zusammensetzungen, in denen der wirkstoffhaltige Kern von einer magensaftresistenten Phase umgeben ist. Gemäß Beispiel 2 kann der Kern geringe Mengen Hydroxypropylcellulose enthalten. Der Wirkstoff soll nach Auflösung der magensaftresistenten Phase schnell freigesetzt werden.

Die EP 0 485 840 A2 offenbart eine orale Arzneiform, die ein den Wirkstoff umschließendes Hüllmaterial aus einem Polysaccharid und einem filmbildenden Polymermaterial enthält.

Ein Nachteil bei dem im Stand der Technik bekannten pharmazeutischen Formulierungen besteht darin, daß der Wirkstoff auch in den Blutkreislauf aufgenommen wird. Diese Menge an Wirkstoff fehlt somit im Darm, so daß die wirksame Dosis des Medikaments um den Teil des Wirkstoffes, der sich im Blut befindet, herabgesetzt wird.

Darüber hinaus müssen Patienten, die. unter intestinalen Erkrankungen leiden, häufig auch nach dem Abklingen der akuten Erkrankung über längere Zeiträume mit dem Wirkstoff, oder Derivaten davon, weiterbehandelt werden, um ein Wiederaufflammen der Erkrankung oder aus der ursprünglichen Erkrankung entstehende Folgeerkrankungen zu verhindern. Bei einer solchen Langzeitbehandlung erweist es sich aber als problematisch, daß eine gewisse Nephrotoxizität von systemisch verfügbarer, also in der Blutbahn befindlicher 5-ASA oder Derivaten davon nicht ausgeschlossen werden kann (M. Barry, Prescribers Journal, 1992, 32, 205).

Es ist somit eine Aufgabe der vorliegenden Erfindung, eine oral verabreichbare pharmazeutische Formulierung zur Verfügung zu stellen, die diese Nachteile nicht aufweist. Erfindungsgemäß werden daher Formulierungen zur Verfügung gestellt, die ein kontrolliertes Freigabeprofil aufweisen, das zu einer hohen lokalen Wirkstoffkonzentration am Wirkort führt und gleichzeitig einen möglichst geringen Blutspiegel des Wirkstoffs gewährleistet.

Im Rahmen der vorliegenden Erfindung wurde nun gefunden, daß sich hierfür Pellet-Formulierungen besonders eignen, da sie im Gegensatz zu einer Tablette die Arzneiform reproduzierbar über weite Bereiche des Darms ausbreiten und so für eine Behandlung entzündlicher Prozesse, die oft größere Abschnitte des Intestinaltraktes befallen, besonders geeignet sind. Um die notwendige lokale Wirkstoffkonzentration zu erreichen, muß der Wirkstoff am Entzündungsort dabei innerhalb relativ kurzer Zeit (bis zu wenigen Stunden) freigesetzt werden, ohne daß er jedoch praktisch sofort freigesetzt würde, damit seine Wirkung nicht zu schnell nachläßt.

Die Verwendung einer quellbaren, gelbildenden Matrix wie in FR-A 2 692 484 beschrieben, eignet sich für Pellets mit einem Durchmesser ≤ 3 mm nicht, da aufgrund des geringen Durchmessers das Polymer sehr schnell vom Wasser durchdrungen wird, dadurch erodiert, und der Wirkstoff damit praktisch sofort (ca. 30 min) freigesetzt werden würde.

Im Rahmen der vorliegenden Erfindung wurde demgegenüber überraschenderweise gefunden, daß, wenn der Wirkstoff im Pellet-Kern in einer im Intestinaltrakt im wesentlichen unlöslichen, für Intestinalflüssigkeiten und den Wirkstoff permeablen nicht-gebildenden Polymermatrix vorliegt, eine im Vergleich zu bereits im Stand der Technik bekannten Aminosalicylsäure-Formulierungen deutlich erniedrigte Abgabe des Wirkstoffes ins Blut, bei gleichzeitig erhöhter lokaler Konzentration des Wirkstoffes am Erkrankungsort im Darm gewährleistet wird.

Gegenstand der vorliegenden Erfindung ist somit eine oral verabreichbare pharmazeutische Pellet-Formulierung mit kontrolliertem Freigabeprofil zur Behandlung des Intestinaltraktes, die einen Kern und einen magensaftresistenten Überzug sowie gegebenenfalls weitere pharmazeutisch verträgliche Zusatzstoffe umfaßt, wobei der Kern als pharmazeutischen Wirkstoff Aminosalicylsäure oder ein pharmazeutisch verträgliches Salz oder Derivat davon einschließt, dadurch gekennzeichnet, daß der Wirkstoff im Kern in einer im Intestinaltrakt im wesentlichen unlöslichen, für Intestinalflüssigkeiten und den Wirkstoff permeablen nichtgelbildenden Polymermatrix vorliegt, wobei das matrixbildende Polymer mindestens 1 Gew.-% des Gesamtgewichts des Kerns ausmacht.

Gegenstand der Erfindung ist weiterhin ein Verfahren zur Herstellung der oben beschriebenen Pellets und deren Verwendung zur Herstellung eines Medikaments zur Behandlung intestinaler Erkrankungen, wie entzündlichen Darmerkrankungen, bevorzugt in der aktiven Phase und/oder in der Remissionsphase, zur Prävention dieser Erkrankungen, zur Verhinderung des Wiederauftretens dieser Erkrankungen oder daraus entstehender Folgeerkrankungen sowie möglicher Begleiterkrankungen sowie zur Behandlung von Darmkrebs. Das Medikament eignet sich insbesondere zur Behandlung von entzündlichen Darmerkrankungen wie Morbus Crohn und Colitis ulcerosa, sowie zur Prävention, Behandlung und/oder Verhinderung des Wiederentstehens von Polypen im Gastrointestinaltrakt. Darüber hinaus eignet sich das Medikament zur Prävention von kolorektalen Karzinomen bei Patienten mit Adenomen und/oder polypösem Wachstum, insbesondere mit polypösem Wachstum im Darm. Außerdem dient das Medikament zur Senkung der Rezidivrate kolorektaler Polypen.

Als Wirkstoffe werden 5-Aminosalicylsäure (auch Mesalazin genannt), 4-Aminosalicylsäure und als Prodrug für 5-ASA dienende 2-Hydroxy-5-phenylazobenzoesäure-Derivate wie Sulfasalazin (5-[4-(2-Pyridylsulfamoyl)phenylazo)salicylsäure) und Balsalazid (das Natriumsalz des Azo-Derivats aus 4-Aminobenzoyl-ß-alanin und 5-Aminosalicylsäure) bevorzugt. Besonders bevorzugt ist 5-ASA.

Der Pellet-Kern umfaßt neben dem Wirkstoff mindestens 1 Gew.-% bezogen auf das Gesamtgewicht des Kerns eines matrixbildenden, im Intestinaltrakt im wesentlichen unlöslichen, für Intestinalflüssigkeiten und den Wirkstoff permeablen nichtgelbildenden Polymers. Als matrixbildende Polymere eignen sich beispielsweise solche Polymere, die im Stand der Technik als Überzugslacke für Arzneimittel mit verzögerter Freisetzung bekannt sind, wie beispielsweise (Meth)acrylestercopolymere.

Unter den im Intestinaltrakt im wesentlichen unlöslichen, für Intestinalflüssigkeiten und den Wirkstoff permeablen Polymeren sind solche bevorzugt, die im Intestinaltrakt unlöslich oder besonders bevorzugt wasserunlöslich sind.

Besonders bevorzugt sind Methylacrylatcopolymere und Ammoniomethacrylatcopolymere der Art, wie sie unter der Handelsbezeichnung Eudragit RS/RL/NE bezogen werden können. Diese Polymere weisen als funktionelle Gruppen Ester-(Eudragit® NE) bzw. Ammoniumgruppen (Eudragit® RL/RS) auf. Bevorzugt werden Poly(ethylacrylat, methylmethacrylat) und Poly(ethylacrylat, methylmethacrylat, trimethylammonioethylmethacrylat-chlorid). Diese Polymere sind zum Beispiel als Poly(ethylacrylat, methylmethacrylat) 2:1 in 40%iger wässeriger Dispersion als Eudragit NE 40 D und als Poly (ethylacrylat, methylmethacrylat, trimethyl ammonioethylmethacrylat-chlorid) 1:2:0,1 in 12,5%iger isopropanolischer Lösung als Eudragit® RS 12,5 und in der Zusammensetzung 1:2:0,2 als Eudragit® RL 12,5 erhältlich. Am bevorzugtesten ist Eudragit® NE 40 D.

Das Polymer muß in einer Menge vorliegen, die ausreicht, eine Matrix für den Wirkstoff zu bilden, und die eine verzögerte Freisetzung des Wirkstoffs gewährleistet. Hierzu hat sich eine Menge von mindestens 1 Gew.-%, bevorzugt mindestens 4 Gew.-%, bezogen auf das Gesamtgewicht des Kerns als geeignet erwiesen. Größere Mengen von z.B. etwa 21 Gew.-% können ebenfalls eingesetzt werden. Bevorzugt werden 4 Gew.-% bis 10 Gew.-% eingesetzt.

Der Wirkstoff ist in der oben beschriebenen Matrix bevorzugt homogen verteilt und wird nach Auflösen des magensaftresistenten Überzugs verzögert freigesetzt. Vorteilhaft erstreckt sich die Matrix mit dem darin homogen verteilten Wirkstoff durch den gesamten Kern.

Der magensaftresistente Überzug soll sich erst nachdem die Formulierung den Magen verlassen hat auflösen. Hierzu notwendige Überzüge sind im Stand der Technik bekannt (z.B. EP 0 453 001 A1).

Erfindungsgemäß bevorzugte magensaftresistente Überzüge umfassen ein Methacrylsäurecopolymer oder Methylhydroxypropylcellulosephthalat. Bevorzugt sind Poly(methacrylsäure, methylmethacrylate), die unter dem Handelsnamen Eudragit® L oder S erhältlich sind und als funktionelle Gruppen freie Carboxylgruppen aufweisen. Diese Polymere sind im Magensaft unlöslich, lösen sich aber in Verdauungssäften in Abhängigkeit von der Anzahl funktioneller Carboxylgruppen oberhalb pH 5,5 - 7. Besonders bevorzugt werden Poly(methacrylsäure, methylmethacrylat) 1:1 (Eudragit® L 100; methacrylic acid copolymer, USP/NF Typ A) und Poly(methacrylsäure, methylmethacrylat) 1:2 (Eudragit® S; methacrylic acid copolymer, USP/NF Typ B). Am bevorzugtesten ist Eudragit® L 100. Mischungen der genannten Überzugsmaterialien insbesondere aus Eudragit® L und Eudragit® S können ebenfalls verwendet werden.

Die Pellet-Formulierung kann einen oder mehrere Überzüge umfassen, allerdings werden Pellet-Formulierungen in denen das Pellet nur einen Überzug umfaßt bevorzugt.

Sowohl der Kern als auch der Überzug der erfindungsgemäßen Pellet-Formulierung können ein oder mehrere der oben genannten Matrix- bzw. Überzugsmaterialen einschließen.

Die erfindungsgemäßen Pellet-Formulierungen können außerdem sowohl im Kern als auch im Überzug weitere pharmazeutisch verträgliche Zusatzstoffe enthalten. Beispiele für pharmazeutisch verträgliche Zusatzstoffe umfassen Polyvinylpyrrolidon, mikrokristalline Cellulose, Siliciumdioxid, Magnesiumstearat, Lactose, Maisstärke, Triethylcitrat, Talk, Titandioxid und Polyethylenglykol.

Eine besonders bevorzugte Pellet-Formulierung gemäß der vorliegenden Erfindung umfaßt im Kern als Wirkstoff 5-ASA in einer Poly(ethylacrylat, methylmethacrylat) 2:1-Matrix, wobei das Polymer als funktionelle Gruppen Estergruppen enthält, und einen magensaftresistenten Überzug, der Poly(methacrylsäure, methylmethacrylat) 1:1 oder 1:2 mit freien Carboxylgruppen als funktionelle Gruppen enthält, sowie gegebenenfalls weitere pharmazeutisch verträgliche Zusatzstoffe.

Darüber hinaus wird eine Mischung aus Eudragit® S und Eudragit® L, bevorzugt etwa 1:1, vorteilhaft in einem Überzug für die erfindungsgemäßen Pellet-Formulierungen eingesetzt.

Die erfindungsgemäße Pellet-Formulierung zeichnet sich durch ein kontrolliertes Freigabeprofil aus. Bevorzugt beträgt die Wirkstofffreigabe in 0,1 M HCl nach 2 h < 10%, insbesondere < 5%, und in künstlichem Darmsaft bei pH 6,8 nach 0,5 h 10-30%, insbesondere 10-20%, nach 2 h 40-60%, insbesondere 40-50%, und nach 6 h mindestens 80%, insbesondere mindestens 85%.

Die erfindungsgemäßen Pellet-Formulierungen können nach herkömmlichen, dem Fachmann bekannten Verfahren, hergestellt werden. Beispielsweise wird zunächst das Matrixmaterial mit dem Wirkstoff und den gegebenenfalls weiteren pharmazeutisch verträglichen Zusatzstoffen gemischt und zu Pellets geformt. Anschließend wird der Überzug in Form einer Lacksuspension in einem geeigneten Suspensionsmittel wie Ethanol und/oder Wasser aufgetragen, z.B. aufgesprüht. Die Pellets können dabei eine Größe von 0,1-3 mm, bevorzugt 0,5 - 1 mm aufweisen und für die Herstellung eines Medikaments in Einheitsdosisformen wie Tabletten oder Kapseln vereinigt werden. Die vorliegende Erfindung betrifft daher auch pharmazeutische Formulierungen, die die erfindungsgemäßen Pellets umfassen, insbesondere Gelatine-Kapseln, die die erfindungsgemäßen Pellets enthalten.

Die so erhaltenen Pellet-Formulierungen führen bei oraler Verabreichung im Vergleich zu anderen Präparaten mit gleichem Wirkstoff zu niedrigeren Wirkstoffkonzentrationen im Blut bei gleichzeitig höherer Konzentration der Wirkstoffe im Darm, wodurch das Nebenwirkungspotential, hervorgerufen durch den systemisch verfügbaren Wirkstoff oder dessen Metaboliten, deutlich herabgesetzt wird.

Damit eignet sich die erfindungsgemäße Pellet-Formulierung besonders für die Behandlung intestinaler Zustände, in denen eine längerfristige Applikation des Wirkstoffs angezeigt ist, wie entzündliche Darmerkrankungen in ihrer aktiven Phase und ihrer Remissionsphase, bei der Prävention von Adenomen und/oder Polypenentstehung, bei der Verhinderung des Rezidivs von Adenomen und/oder Polypen und bei der Verhinderung daraus entstehender Folgeerkrankungen und möglicher Begieiterkrankungen.

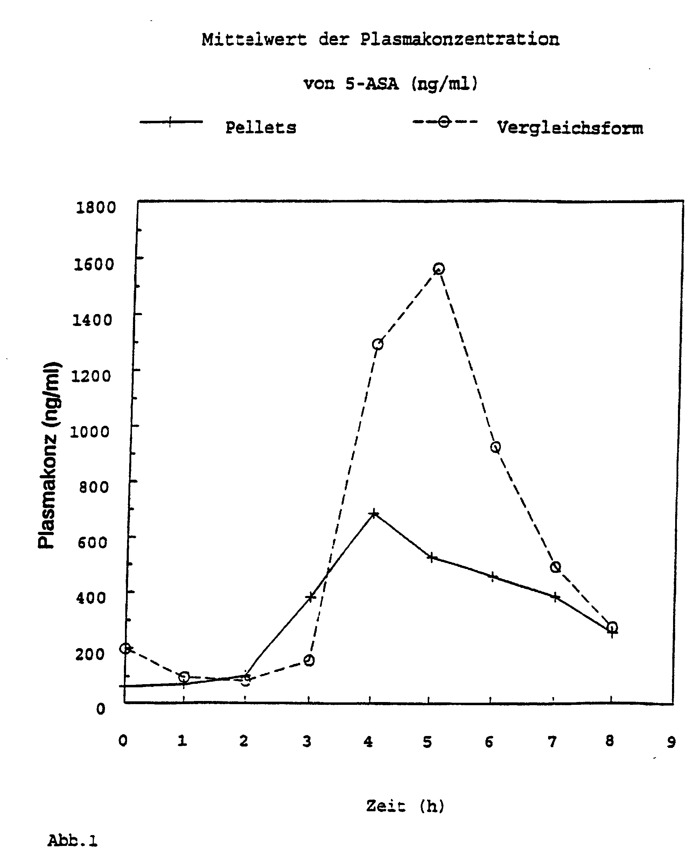

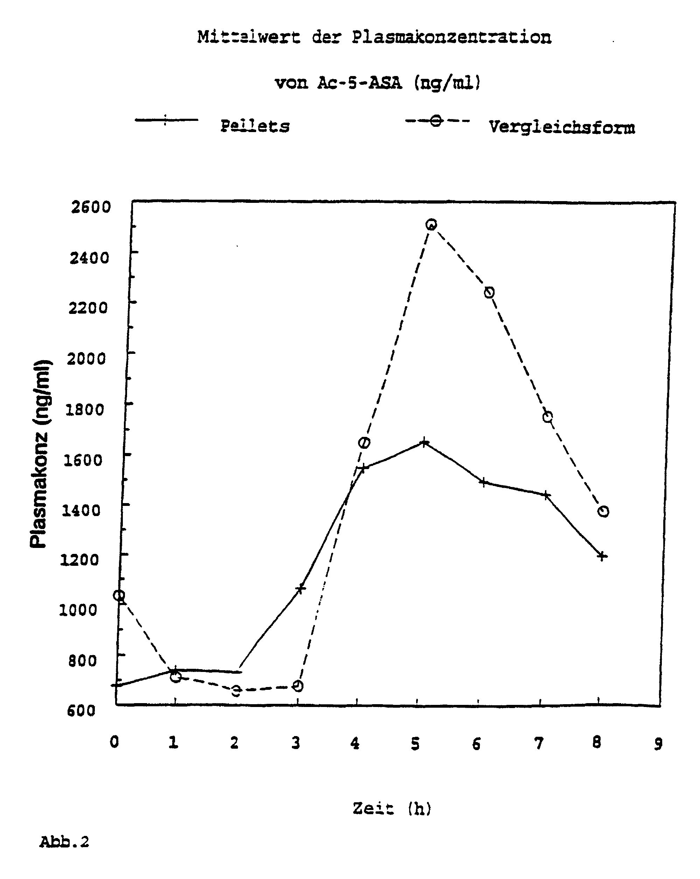

Die Abbildungen 1 und 2 zeigen Diagramme der Plasmakonzentrationen von 5-ASA (Abb. 1) und Ac-5-ASA (Abb. 2) gegen die Zeit.

Die folgenden Beispiele dienen zur Veranschaulichung der Erfindung.

Beispiel 1 beschreibt zwei verschiedene Pellet-Kerne (Beispiel 1.1-1.2) und vier verschiedene Pellet-Überzüge (Beispiel 1.a-1.d). Die verschiedenen Kerne können beliebig mit den verschiedenen Überzügen kombiniert werden, wobei der Pellet-Kern aus Beispiel 1.1 zusammen mit dem Überzug aus Beispiel 1.a ein besonders bevorzugtes Beispiel darstellt.

### Beispiel 1

Beispiele für Pellet-Kerne:

### 1.1

| | | |
|---|---|---|
| I | Mesalazin | 5000 g |
| II | Cellulose, mikrokristallin | 1500 g |
| III | Hydroxypropylmethylcellulose | 200 g |
| IV | Siliciumdioxid | 25 g |
| V | Poly(ethylacrylat, methylmethacrylat) 2:1 als 40%ige wässerige Dispersion, Handelsbezeichnung Eudragit® NE 40 D | 750 g |
| VI | Magnesiumstearat | 250 g |

I-IV werden gemischt, mit V befeuchtet und intensiv geknetet. Zum Schluß wird VI eingestreut. Die feuchte Masse wird durch einen Extruder mit der Matrizenbohrung 1 mm gedrückt. Die extrudierten Stränge werden in ca. 1 mm lange Stücke geschnitten und in einem Spheronizer gerundet. Bei 60°C werden die Pellets getrocknet.

### 1.2

| | | |
|---|---|---|
| I | Mesalazin | 5000 g |
| II | Cellulose, mikrokristallin | 1500 g |
| III | Hydroxypropylmethylcellulose | 200 g |
| IV | Siliciumdioxid | 25 g |
| V | Poly(ethylacrylat, methylmethacrylat,trimethylammonioethylmethacrylat-chlorid) 1:2:0,1; als 12,5%ige isopropanolische Lösung; Handelsbezeichnung Eudragit® RS 12,5 | 2500 g |
| VI | Magnesiumstearat | 250 g |

I-IV werden gemischt, mit V befeuchtet und intensiv geknetet. Zum Schluß wird VI eingestreut. Die feuchte Masse wird durch einen Extruder mit der Matrizenbohrung 1 mm gedrückt. Die extrudierten Stränge werden in ca. 1 mm lange Stücke geschnitten und in einem Spheronizer gerundet. Bei 60°C werden die Pellets getrocknet.

### Beispiele für Pellet Überzüge

### Rezepturen für 5000 g Pellets, entsprechend den Beispielen 1.1-1.2

### 1.a

| | |
|---|---|
| I Poly(methacrylsäure, methylmethacrylat) 1:1; Handelsname Eudragit® L 100; (Methacrylic acid copolymer, USP/NF Typ A) | 750 g |
| II Triethylcitrat | 75 g |
| III Talk | 200 g |
| IV Titandioxid | 125 g |
| V Magnesiumstearat | 50 g |

I wird in 7000 g eines Ethanol/Wasser-Gemisches (8:2) gelöst. II-V werden in der Lösung suspendiert; in einer geeigneten Apparatur wird die Lacksuspension bei einer Zulufttemperatur von 40°C aufgesprüht.

### 1.b

| | | |
|---|---|---|
| I | Poly(methacrylsäure, methylmethacrylat) 1:2 ; Handelsname Eudragit® S; (Methacrylic acid copolymer, USP/NF Typ B) | 350 g |
| II | Triethylcitrat | 35 g |
| III | Talk | 100 g |
| IV | Titandioxid | 125 g |
| V | Magnesiumstearat | 50 g |

I wird in 3500 g eines Ethanol/Wasser-Gemisches (8:2) gelöst. II-V werden in der Lösung suspendiert; in einer geeigneten Apparatur wird die Lacksuspension bei einer Zulufttemperatur von 40°C aufgesprüht.

### 1.c

| | | |
|---|---|---|
| I | [Poly(methacrylsäure, methylmethacrylat) 1:2; Handelsname Eudragit® S; (Methacrylic acid copolymer, USP/NF Typ B) Poiy(methacrylsäure, methylmethacrylat) 1:1; Handelsname Eudragit® L 100; (Methacrylic acid copolymer, USP/NF Typ A)] (Im Verhältnis 1,1:1 gemischt) | 420 g |
| II | Triethylcitrat | 75 g |
| III | Talk | 200 g |
| IV | Titandioxid | 125 g |
| V | Magnesiumstearat | 50 g |

I wird in 5000 g eines Ethanol/Wasser-Gemisches (8:2) gelöst. II-V werden in der Lösung suspendiert; in einer geeigneten Apparatur wird die Lacksuspension bei einer Zulufttemperatur von 40°C aufgesprüht.

### 1.d

| | | |
|---|---|---|
| I | Methylhydroxypropylcellulosephthalat | 410 g |
| II | Ethylcellulose | 44 g |
| III | Polyethylenglykol 6000 | 40 g |
| IV | Talk | 200 g |
| V | Titandioxid | 125 g |
| VI | Magnesiumstearat | 50 g |

I und II werden in 5000 g eines Ethanol/Wasser-Gemisches (9:1) gelöst. III-VI werden in der Lösung suspendiert; in einer geeigneten Apparatur wird die Lacksuspension bei einer Zulufttemperatur von 40°C aufgesprüht.

### Beispiel 2

Zur Bestimmung der Freisetzung des Wirkstoffs aus den erfindungsgemäßen Pellets wurde die "Basket"-Methode verwendet. Die Rührergeschwindigkeit betrug 100 Umdrehungen/min, die Temperatur wurde bei 37°C konstant gehalten. Als künstlicher Magensaft wurde gemäß USP 0,1 M HCl verwendet als künstlicher Darmsaft USP-Phosphatpuffer (pH 6,8).

In Tabelle 1 ist die Wirkstofffreisetzung der erfindungsgemäßen Pellet-Formulierung mit einem Kern nach Beispiel 1.1 und einem Überzug nach Beispiel 1.a unter den obengenannten Bedingungen angegeben.

**Tabelle 1**

| pH-Wert | Zeit [min] | Freisetzung [%] |
|---|---|---|
| 1,2 | 120 | 1,6 |
| | | |
| | 30 | 12,2 |
| | 60 | 24,9 |
| | 90 | 36,0 |
| | 120 | 45,2 |
| 6,8 | 150 | 53,0 |
| | 180 | 59,7 |
| | 240 | 70,6 |
| | 300 | 78,8 |
| | 360 | 85,4 |

### Beispiel 3

Um Ergebnisse über die Wirkstoffaufnahme in das Blut nach Verabreichung der erfindungsgemäßen Pellet-Formulierung zu erhalten, wurden die Plasmakonzentrationen von 5-ASA und Acetyl-5-ASA (Ac-5-ASA), seinem Abbauprodukt, zeitabhängig untersucht. 24 gesunde Probanden erhielten im Rahmen einer cross-over Anordnung 500 mg 5-ASA in zwei unterschiedlichen galenischen Formulierungen (erf. Pellets mit Kern nach Beispiel 1.1 und Überzug nach Beispiel 1.a und im Handel erhältliche Salofalk®-Tabletten (Mesalazin in Form magensaftresistenter Tabletten) als Vergleichspräparat) über einen Zeitraum von 4 Tagen (3 x 500 mg 5-ASA täglich). Zur Bestimmung der Plasmakonzentrationen von 5-ASA und Acetyl-5-ASA wurden den Probanden venöse Blutproben entnommen.

In Tab. 2 ist die aus 24 Patienten gemittelte Plasmakonzentration von 5-ASA und Ac-5-ASA unter steady-state-Bedingungen dargestellt.

**Tabelle 2**

| Plasmakonzentration von 5-ASA und Ac-5-ASA in 24 Probanden [ng/ml] (Mittelwert) | | | | |
|---|---|---|---|---|
| | Pellets | | Vergleichspräparat | |
| Zeit nach Verabreichung [h] | [5-ASA] | [Ac-5-ASA] | [5-ASA] | [Ac-5-ASA] |
| 0 | 63,49 | 676,35 | 198,42 | 1033,89 |
| 1 | 71,50 | 739,46 | 96,66 | 711,70 |
| 2 | 102,97 | 731,34 | 82,86 | 657,16 |
| 3 | 382,02 | 1063,59 | 156,55 | 675,83 |
| 4 | 686,03 | 1549,00 | 1293,30 | 1651,01 |
| 5 | 527,39 | 1653,73 | 1564,33 | 2511,99 |
| 6 | 456,70 | 1493,00 | 924,75 | 2243,11 |
| 7 | 384,25 | 1442,96 | 492,91 | 1755,05 |
| 8 | 257,16 | 1196,51 | 275,11 | 1377,46 |

Aus Tabelle 2 und aus den Abbildungen 1 und 2 ist ersichtlich, daß sowohl von 5-ASA als auch von seinem Metaboliten Ac-5-ASA deutlich geringere Plasmaspiegel erreicht werden können, wenn den Probanden 5-ASA in Form der erfindungsgemäßen Pellet-Formulierung verabreicht wird. Dieses Ergebnis wird durch die durchschnittlichen Cₘₐₓ-Werte (Durchschnitt der aus den Daten der einzelnen Probanden errechneten Cₘₐₓ-Werte) bestätigt. Der durchschnittliche Cₘₐₓ-Wert betrug bei der Vergleichsformulierung 2001 ng/ml für 5-ASA und 2617 ng/ml für Ac-5-ASA während der durchschnittliche Cₘₐₓ-Wert bei der erfindungsgemäßen Pellet-Formulierung bei 755 ng/ml für 5-ASA und 1810 ng/ml für Ac-5-ASA lag. Damit liegt der durchschnittliche Cₘₐₓ-Wert der Pellet Formulierung bei nur 37,7 % des durchschnittlichen Cₘₐₓ-Wertes der Vergleichsformulierung für 5-ASA und bei nur 69 % für Ac-5-ASA.

### Beispiel 4

Um eine erhöhte lokale Freisetzung der 5-ASA im Darm zu beweisen, wurden in einer weiteren Untersuchung Faecesproben von 4 Probanden, die 1500 mg 5-ASA erhalten hatten, auf 5-ASA und Ac-5-ASA hin untersucht. Dazu wurde der Faeces der Probanden über 71 Stunden gesammelt und auf freies, nicht im Pellet oder in der Vergleichsformulierung gebundenes, 5-ASA und Ac-5-ASA untersucht (Tab. 3). Als erfindungsgemäße Pellets wurden solche mit einem Kern nach Beispiel 1.1 und einem Überzug nach Beispiel 1.a eingesetzt, als Vergleichspräparat dienten im Handel erhältliche Salofalk®-Tabletten (Mesalazin in Form magensaftresistenter Tabletten).

**Tabelle 3**

| Kummulative fäkale Ausscheidung von 5-ASA und 5-Ac-ASA von 4 Probanden [mg] (Mittelwert) | | | |
|---|---|---|---|
| Pellets | | Vergleichspräparat | |
| [5-ASA], | [Ac-5-ASA] | [5-ASA] | [Ac-5-ASA] |
| 287,5 | 367,9 | 222,9 | 275,7 |

Aus der Tabelle ist ersichtlich, daß sowohl die Menge an der durch die erfindungsgemäße Pellet-Formulierung im Darm freigesetzten 5-ASA mit 287,5 mg als auch die Menge an freier Ac-5-ASA mit 367,9 mg um 29 % bzw. 44,4 % höher ist als bei der Vergleichsformulierung. Da Ac-5-ASA im Darm nur durch die Wechselwirkung mit der Darmschleimhaut entstehen kann, zeigt die erhöhte Menge an Ac-5-ASA bei der Pellet-Formulierung, daß deutlich mehr Wirkstoff mit der Darmschleimhaut in Kontakt kommt und damit seine heilende Wirkung entfalten kann, als bei der Vergleichsformulierung.

Diese Untersuchungen belegen, daß durch die erfindungsgemäße Pellet-Formulierung die Konzentration des Wirkstoffes 5-ASA bzw. dessen Abbauproduktes Ac-5-ASA im Vergleich zu handelsüblichen 5-ASA-Präparaten im Blut wesentlich erniedrigt wird und damit auch die Gefahr von eventuellen Nebenwirkungen (Nephrotoxizität etc.) geringer ist. Als Folge der erniedrigten Aufnahme des Wirkstoffes ins Blut, stehen deutlich höhere Mengen des Wirkstoffes im Darm zur Verfügung. Diese kommen auch mit der Darmschleimhaut in Kontakt und können dort ihre Wirkung entfalten, wie die im Vergleich zur Vergleichsformulierung höheren Mengen an Ac-5-ASA, das im Darm durch den direkten Kontakt von 5-ASA mit der Darmschleimhaut entsteht, beweisen. Im Gegensatz zum systemisch verfügbaren 5-ASA und Ac-5-ASA kann das im Darm vorhandene 5-ASA und Ac-5-ASA nicht nephrotoxisch wirken, da es nicht über die Niere, sondern mit dem Faeces ausgeschieden wird.

Damit eignet sich die erfindungsgemäße Pellet-Formulierung bevorzugt für intestinale Zustände in denen eine längerfristige Applikation des Wirkstoffes angezeigt ist, wie entzündliche Darmerkrankungen in ihrer aktiven Phase und in ihrer Remissionsphase, bei der Prävention von Polypenentstehung, bei der Verhinderung des Rezidivs von Polypen und bei der Verhinderung daraus entstehender Folgeerkrankungen und möglicher Begleiterkrankungen.

## Patentansprüche

1. Oral verabreichbare pharmazeutische Pellet-Formulierung mit kontrolliertem Freigabeprofil zur Behandlung des Intestinaltraktes, die einen Kern und einen magensaftresistenten Überzug sowie gegebenenfalls weitere pharmazeutisch verträgliche Zusatzstoffe umfaßt, wobei der Kern als pharmazeutischen Wirkstoff Aminosalicylsäure oder ein pharmazeutisch verträgliches Salz oder Derivat davon einschließt, **dadurch gekennzeichnet, daß** der Wirkstoff im Kern in einer im Intestinaltrakt im wesentlichen unlöslichen, für Intestinalflüssigkeiten und den Wirkstoff permeablen nichtgelbildenden Polymermatrix vorliegt, wobei das matrixbildende Polymer mindestens 1 Gew.-% des Gesamtgewichts des Kerns ausmacht.

2. Pellet-Formulierung nach Anspruch 1, **dadurch gekennzeichnet, daß** das matrixbildende Polymer ausgewählt ist aus der Gruppe bestehend aus Poly(ethylacrylat, methylmethacrylat) und Poly(ethylacrylat, methylmethacrylat, trimethylammonioethyl methacrylat-chlorid) .

3. Pellet-Formulierung nach einem der vorhergehenden Ansprüche, **dadurch gekennzeichnet, daß** das matrixbildende Polymer 4 - 10 Gew.-% des Gesamtgewichts des Kerns ausmacht.

4. Pellet-Formulierung nach einem der vorhergehenden Ansprüche, **dadurch gekennzeichnet, daß** sich die Polymermatrix durch den gesamten Kern erstreckt und der Wirkstoff in der Polymermatrix homogen verteilt vorliegt.

5. Pellet-Formulierung nach einem der vorhergehenden Ansprüche, **dadurch gekennzeichnet, daß** der Wirkstoff 5-Aminosalicylsäure ist.

6. Pellet-Formulierung nach einem der vorhergehenden Ansprüche, **dadurch gekennzeichnet, daß** der magensaftresistente Überzug ein Methacrylsäurecopolymer oder Methylhydroxypropylcellulosephthalat umfaßt.

7. Pellet-Formulierung nach Anspruch 6, **dadurch gekennzeichnet, daß** der magensaftresistente Überzug Poly(methacrylsäure, methylmethacrylat) umfaßt, wobei das Polymer als funktionelle Gruppen freie Carboxylgruppen enthält.

8. Pellet-Formulierung nach einem der vorhergehenden Ansprüche, **dadurch gekennzeichnet, daß** das Pellet nur eine Überzugschicht umfaßt.

9. Pellet-Formulierung nach einem der vorhergehenden Ansprüche, umfassend als Wirkstoff 5-Aminosalicylsäure in einer Poly(ethylacrylat, methylmethacrylat) 2:1-Matrix, wobei das Polymer als funktionelle Gruppen Estergruppen enthält, im Kern und einen magensaftresistenten Überzug, der Poly(methacrylsäure, methylmethacrylate) 1:1 oder 1:2, wobei das Polymer als funktionelle Gruppen freie Carboxylgruppen enthält, umfaßt, sowie gegebenenfalls weitere pharmazeutisch verträgliche Zusatzstoffe.

10. Pellet-Formulierung nach einem der vorhergehenden Ansprüche, **dadurch gekennzeichnet, daß** die Wirkstofffreigabe in 0,1 M HCl nach 2 h < 10 % und in künstlichem Darmsaft bei pH = 6,8 nach 0,5 h 10 - 30 %, nach 2 h 40 - 60 % und nach 6 h mindestens 80 % beträgt.

11. Pharmazeutische Formulierung, **dadurch gekennzeichnet, daß** sie Pellets nach einem der Ansprüche 1 bis 10 umfaßt.

12. Pharmazeutische Formulierung nach Anspruch 11, **dadurch gekennzeichnet, daß** es sich um eine Gelatine-Kapsel oder eine Tablette handelt, die die Pellets nach einem der Ansprüche 1 bis 10 enthält.

13. Verwendung der Pellet-Formulierung nach einem der vorhergehenden Ansprüche zur Herstellung eines Medikaments zur Behandlung von entzündlichen Darmerkrankungen, zur Prävention dieser Erkrankungen, zur Verhinderung des Wiederauftretens dieser Erkrankungen oder daraus entstehender Folgeerkrankungen sowie möglicher Begleiterkrankungen sowie zur Behandlung von Darmkrebs.

14. Verwendung nach Anspruch 13, worin das Medikament zur Behandlung von entzündlichen Darmerkrankungen in der aktiven Phase und/oder Remissionsphase dient.

15. Verwendung nach Anspruch 13, worin das Medikament zur Prävention, Behandlung und/oder Verhinderung des Wiederentstehens von Polypen im Gastrointestinaltrakt dient.

16. Verwendung nach Anspruch 15, worin das Medikament zur Prävention von kolorektalen Karzinomen bei Patienten mit Adenomen und/oder polypösem Wachstum, insbesondere mit polypösem Wachstum im Darm, dient.

17. Verwendung nach Anspruch 15, worin das Medikament zur Senkung der Rezidivrate von Adenomen und/oder kolorektaler Polypen dient.

18. Verfahren zur Herstellung einer oral verabreichbaren Pellet-Formulierung nach einem der Ansprüche 1-10, worin das matrixbildende Polymer mit dem Wirkstoff und den gegebenenfalls weiteren pharmazeutisch verträglichen Zusatzstoffen gemischt und zu Pellets geformt wird und anschließend der Überzug in Form einer Lacksuspension aufgetragen wird.

## Claims

1. An orally administrable pharmaceutical pellet formulation having a controlled release profile for the treatment of the intestinal tract, which comprises a core and an enteric coating and, if appropriate, further pharmaceutically tolerable additives, the core including, as a pharmaceutical active compound, aminosalicylic acid or a pharmaceutically tolerable salt or derivative thereof, wherein the active compound is present in the core in a non gel-forming polymer matrix which is essentially insoluble in the intestinal tract and permeable to intestinal fluids and the active compound, the matrix-forming polymer making up at least 1% by weight of the total weight of the core.

2. The pellet formulation as claimed in claim 1, wherein the matrix-forming polymer is selected from the group consisting of poly(ethyl acrylate, methyl methacrylate) and poly(ethyl acrylate, methyl methacrylate, trimethylammonioethyl methacrylate chloride).

3. The pellet formulation as claimed in one of the preceding claims, wherein the matrix-forming polymer makes up 4-10% by weight of the total weight of the core.

4. The pellet formulation as claimed in one of the preceding claims, wherein the polymer matrix extends through the entire core and the active compound is homogeneously dispersed in the polymer matrix.

5. The pellet formulation as claimed in one of the preceding claims, wherein the active compound is 5-aminosalicylic acid.

6. The pellet formulation as claimed in one of the preceding claims, wherein the enteric coating comprises a methacrylic acid copolymer or methylhydroxypropylcellulose phthalate.

7. The pellet formulation as claimed in claim 6, wherein the enteric coating contains poly(methacrylic acid, methyl methacrylate), the polymer containing free carboxyl groups as functional groups.

8. The pellet formulation as claimed in one of the preceding claims, wherein the pellet only comprises one coating layer.

9. The pellet formulation as claimed in one of the preceding claims, comprising, as an active compound, 5-aminosalicylic acid in a poly(ethyl acrylate, methyl methacrylate) 2:1 matrix, the polymer containing ester groups as functional groups, in the core and an enteric coating which comprises poly(methacrylic acid, methyl methacrylates) 1:1 or 1:2, the polymer containing free carboxyl groups as functional groups, and also, if appropriate, further pharmaceutically tolerable additives.

10. The pellet formulation as claimed in one of the preceding claims, wherein the release of active compound in 0.1 M HCl after 2 h is < 10% and in artificial gastric juice at pH=6.8 after 0.5 h 10-30%, after 2 h 40-60% and after 6 h at least 80%.

11. A pharmaceutical formulation, which comprises pellets as claimed in one of claims 1 to 10.

12. The pharmaceutical formulation as claimed in claim 11, which is a gelatin capsule or a tablet which contains the pellets as claimed in one of claims 1 to 10.

13. The use of the pellet formulation as claimed in one of the preceding claims for the production of a medicament for the treatment of inflammatory intestinal disorders, for the prevention of these disorders, for the prevention of the recurrence of these disorders or secondary disorders resulting therefrom and also possible accompanying disorders, and for the treatment of intestinal cancer.

14. The use as claimed in claim 13, in which the medicament is used for the treatment of inflammatory intestinal disorders in the active phase and/or the remission phase.

15. The use as claimed in claim 13, in which the medicament is used for the prevention, treatment and/or prevention of the recurrence of polyps in the gastrointestinal tract.

16. The use as claimed in claim 15, in which the medicament is used for the prevention of colorectal carcinomas in patients with adenomas and/or polypous growth, in particular with polypous growth in the intestine.

17. The use as claimed in claim 15, in which the medicament is used for lowering the recurrence rate of adenomas and/or colorectal polyps.

18. A process for the production of an orally administrable pellet formulation as claimed in one of claims 1-10, in which the matrix-forming polymer is mixed with the active compound and, if appropriate, the further pharmaceutically tolerable additives and shaped to give pellets and the coating is then applied in the form of a lacquer suspension.

## Revendications

1. Formulation de pilule pharmaceutique, administrable oralement, et présentant un profil de libération contrôlée pour le traitement du tractus intestinal, qui comporte un noyau et un revêtement résistant au suc gastrique ainsi qu'éventuellement d'autres additifs pharmaceutiquement compatibles, le noyau renfermant, comme substance active pharmaceutique, de l'acide aminosalicylique ou un sel ou dérivé pharmaceutiquement compatible de celui-ci, **caractérisée en ce que** la substance active se présente dans le noyau dans une matrice de polymère sensiblement insoluble, perméable aux liquides intestinaux et à la substance active et non génératrice de gel, le polymère formant la matrice représentant au moins 1 % en poids du poids total du noyau.

2. Formulation de pilule suivant la revendication 1, **caractérisée en ce que** le polymère formant la matrice est choisi parmi le groupe constitué du poly(éthylacrylate, méthylméthacrylate) et du poly(éthylacrylate, méthylméthacrylate, triméthylammonioéthylméthacrylatechlorure).

3. Formulation de pilule suivant l'une des revendications précédentes, **caractérisée en ce que** le polymère formant la matrice représente 4-10% en poids du poids total du noyau.

4. Formulation de pilule suivant l'une des revendications précédentes, **caractérisée en ce que** la matrice de polymère s'étend à travers tout le noyau et **en ce que** la substance active se présente sous une forme répartie de manière homogène dans la matrice de polymère.

5. Formulation de pilule suivant l'une des revendications précédentes, **caractérisée en ce que** la substance active est de l'acide 5-aminosalicylique.

6. Formulation de pilule suivant l'une des revendications précédentes, **caractérisée en ce que** le revêtement résistant au suc gastrique comporte un copolymère d'acide méthacrylique ou du phtalate de méthylhydroxypropylcellulose.

7. Formulation de pilule suivant la revendication 6, **caractérisée en ce que** le revêtement résistant au suc gastrique comporte du poly(acide méthacrylique, méthylméthacrylate), le polymère contenant des groupes carboxyle libres comme groupes fonctionnels.

8. Formulation de pilule suivant l'une des revendications précédentes, **caractérisée en ce que** la pilule ne comporte qu'une couche de revêtement.

9. Formulation de pilule suivant l'une des revendications précédentes, comprenant dans le noyau, comme substance active, de l'acide 5-aminosalicylique dans une matrice de poly(éthylacrylate, méthylméthacrylate) 2/1, le polymère contenant des groupes ester comme groupes fonctionnels, et un revêtement résistant au suc gastrique, qui comporte du poly(acide méthacrylique, méthylméthacrylate) 1/1 ou 1/2, le polymère contenant des groupes carboxyle libres comme groupes fonctionnels, ainsi qu'éventuellement d'autres additifs pharmaceutiquement compatibles.

10. Formulation de pilule suivant l'une des revendications précédentes, **caractérisée en ce que** la libération de substance active est dans du HCI 0,1 M après 2 heures < 10% et dans du suc intestinal artificiel à pH = 6,8 après 0,5 heure de 10-30%, après 2 heures de 40-60% et après 6 heures d'au moins 80%.

11. Formulation pharmaceutique, **caractérisée en ce qu'**elle comporte des pilules suivant l'une des revendications 1 à 10.

12. Formulation pharmaceutique suivant la revendication 11, **caractérisée en ce qu'**il s'agit d'une capsule de gélatine ou d'un comprimé qui contient les pilules suivant une des revendications 1 à 10.

13. Utilisation de la formulation de pilule suivant l'une des revendications précédentes, pour la fabrication d'un médicament destiné au traitement de maladies inflammatoires de l'intestin, pour la prévention de ces maladies, pour empêcher la réapparition de ces maladies et les maladies consécutives qui en résultent ainsi que les troubles collatéraux éventuels, ainsi que pour le traitement du cancer de l'intestin.

14. Utilisation suivant la revendication 13, dans laquelle le médicament sert au traitement de maladies inflammatoires de l'intestin dans la phase active et/ou dans la phase de rémission.

15. Utilisation suivant la revendication 13, dans laquelle le médicament sert à la prévention, au traitement et/ou à l'empêchement de la réapparition de polypes dans le tractus gastro-intestinal.

16. Utilisation suivant la revendication 15, dans laquelle le médicament sert à la prévention de carcinomes colorectaux chez des patients présentant des adénomes et/ou une croissance polypeuse, en particulier une croissance polypeuse dans l'intestin.

17. Utilisation suivant la revendication 15, dans laquelle le médicament sert à diminuer les taux de récidive d'adénomes et/ou de polypes colorectaux.

18. Procédé de préparation d'une formulation de pilule administrable oralement suivant une des revendications 1 à 10, dans lequel le polymère formant la matrice est mélangé avec la substance active et éventuellement les autres additifs pharmaceutiquement compatibles et est amené sous la forme de pilules et en ce qu'ensuite le revêtement est appiqué sous la forme d'une suspension de laque.
